Europäisches Patentamt

European Patent Office

Office européen des brevets

(11) Publication number: **0 279 433 B1**

# EUROPEAN PATENT SPECIFICATION

(45) Date of publication of patent specification: **23.10.91** (51) Int. Cl.⁵: **C12N 15/40**, C12N 15/82

(21) Application number: **88102322.0**

(22) Date of filing: **18.02.88**

(54) Plants resistant to cucumber mosaic virus infection.

(30) Priority: **20.02.87 JP 38288/87**
**25.02.87 JP 43443/87**

(43) Date of publication of application:
**24.08.88 Bulletin 88/34**

(45) Publication of the grant of the patent:
**23.10.91 Bulletin 91/43**

(84) Designated Contracting States:
**AT BE CH DE ES FR GB GR IT LI LU NL SE**

(56) References cited:
**EP-A- 0 223 452**
**WO-A-86/05516**

**CHEMICAL ABSTRACTS, vol. 88, 1978, page
191, abstract no. 17606s, Columbus, Ohio,
US; A.R. GOULD et al.: "Determination of the
sequence homology between the four RNA
species of cucumber mosaic virus by hy-
bridization analysis with complementary
DNA", && NUCLEIC ACIDS RES. 1977, 4(11),
3787-802**

(73) Proprietor: **Takeda Chemical Industries, Ltd.
27, Doshomachi 2-chome Higashi-ku
Osaka-shi Osaka-fu(JP)**

(72) Inventor: **Furusawa, Iwao
1-23, Takano-higashibirakicho
Sakyo-ku Kyoto 606(JP)**
Inventor: **Onda, Haruo
201, 17-1 Namiki 3-chome
Tsukuba Ibaraki 300-42(JP)**
Inventor: **Komiya, Takeya
7-43, Minamisakurazuka 1-chome
Toyonaka Osaka 560(JP)**

(74) Representative: **von Kreisler, Alek,
Dipl.-Chem. et al
Patentanwälte Von Kreisler-Selting-Werner,
Deichmannhaus am Hauptbahnhof
W-5000 Köln 1(DE)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

JOURNAL OF BIOCHEMISTRY, vol. 97, no. 1, 1985, pages 161-171; S. HIDAKA et al.: "Messenger RNA structure participating in the initiation of synthesis of cucumber mosaic virus coat protein"

CHEMICAL ABSTRACTS, vol. 103, 1985, page 388, abstract no. 68384d, Columbus, Ohio, US; J.A. DODDS et al.: "Cross protection between strains of cucumber mosaic virus: effect of host and type of inoculum on accumulation of virions and double-stranded RNA of the challenge strain", VIROLOGY 1985, 144(2), 301-9

SCIENCE, vol. 232, 9th May 1986, pages 738-743; P.P. ABEL et al.: "Delay of disease development in transgenic plants that express the tobacco mosaic virus coat protein gene"

## Description

This invention relates to a DNA coding for the coat protein (hereinafter abbreviated as "CP") of the strain Y of cucumber mosaic virus (hereinafter abbreviated as "CMV"). Said coat protein "CP" is useful in breeding plants resistant to CMV.

A method so far used for breeding plants resistant to plant viruses is the "cross protection" method which comprises infecting plants with attenuated or weak viruses in advance to thereby attain protection from naturally occurring virulent viruses [Annual Review of Phytopathology, 14, 75 (1976); Phytopathology, 57, 1347 (1976); Plant Disease Report, 64, 538 (1980)]. However, this method is known to be disadvantageous in that the attenuated viruses may mutate into more virulent viruses during replication thereof in plant bodies ["Plant-Microbe Interactions: Molecular and Genetic Perspectives", 1, 420 (1984)], that the attenuated viruses may cause exacerbation of diseases in synergy with other viruses [Phytopathology, 57, 232 (1957)], that an attenuated virus to a certain plant may be a virulent virus in another plant or even attenuated viruses may have a certain degree of pathogenicity ["Plant Disease: An Advanced Treatise", 5 279 (1980)]. In addition to the technique of using attenuated viruses, an attempt to cause expression of the gene coding for the coat protein of tobacco mosaic virus (hereinafter abbreviated as "TMV") by introducing into tobacco cells was made very recently with success as technique of breeding tobacco plants resistant to TMV by using the genetic engineering technology [Science, 232, 738 (1986)].

Meanwhile, CMV infection can take place very widely in a total of 45 plant families, inclusive of Cucurbitaceae, Solanaceae, Cruciferae, Compositae, Rosaceae and Liliaceae. In particular, CMV infection of the families Cucurbitaceae, Solanaceae and Cruciferae results in great damages to crops. As regards the strain Q of CMV which is mainly distributed in Europe and America, the base sequence of its CP gene is already known [European Journal of Biochemistry, 126, 217 (1982)]. As regards the strain Y of CMV which is distributed mainly in Japan, however, only part of the amino acid sequence and part of the RNA sequence have been determined [Journal of Biochemistry, 97, 161 (1985)]. No attempts have been made as yet to eradicate the problem caused by the strain Q of CMV or the strain Y, either, from the genetic engineering viewpoint.

As mentioned above, the CP gene of the CMV strain Y (hereinafter abbreviated as "CMV-Y") has not been cloned as yet and remains unknown in many respects. Therefore, development of a method of breeding CMV-resistant plants by cloning the CP gene, determining the base sequence thereof, inserting said gene into a Ti plasmid, a plasmid with the cauliflower mosaic virus gene inserted therein, or the like vector DNA to be used in plant cells, and transforming plant cells with the resultant DNA to thereby allow the CP of CMV-Y to be expressed in said cells, by using genetic engineering techniques has been waited for.

As a result of their intensive investigations in search of a method of breeding CMV-resistant plants, the present inventors found that the cDNA synthesized from RNA3 extracted from CMV-Y contains the base sequence coding for the CP of CMV and further that regenerated plants resistant to CMV can be produced by transformation of plant cells with a vector DNA with a DNA containing said base sequence being inserted therein, followed by callus formation, callus growth, adventitious embryo differentiation or organogenesis if necessary, and plant regeneration. The present invention has been completed on the basis of these findings.

Thus, the invention relates to:

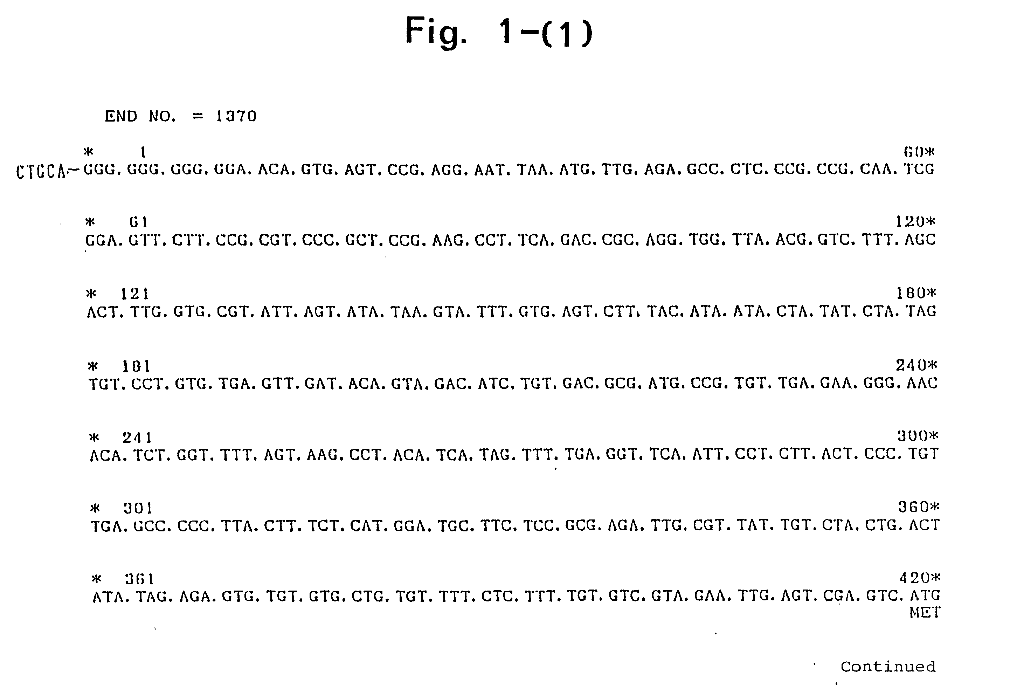

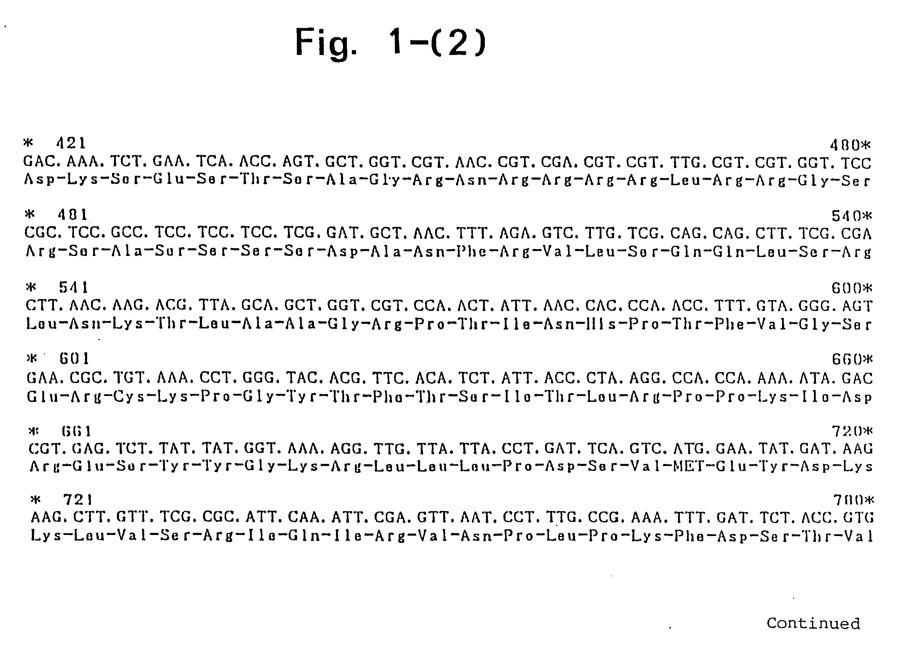

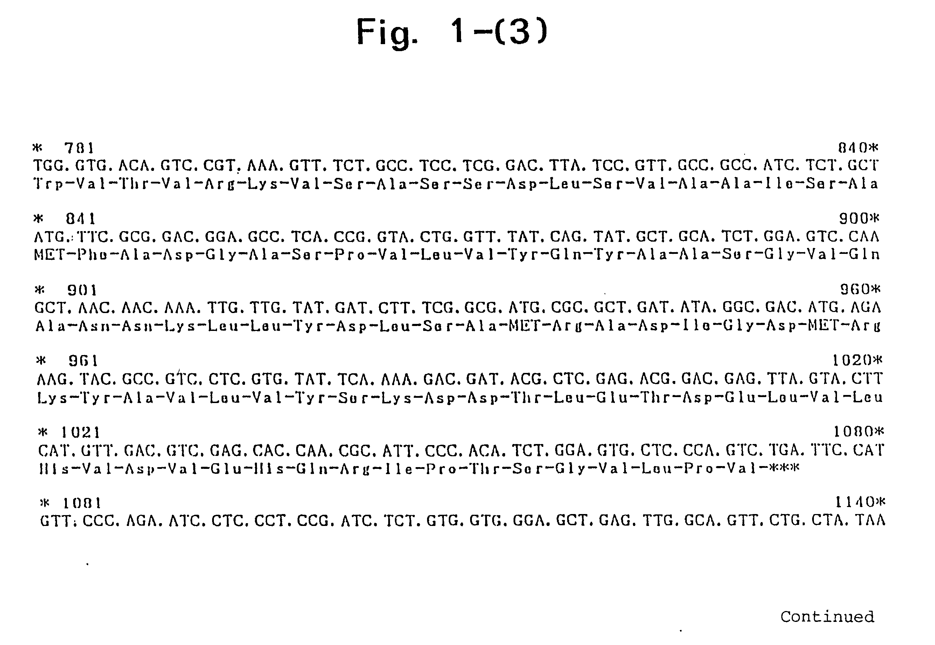

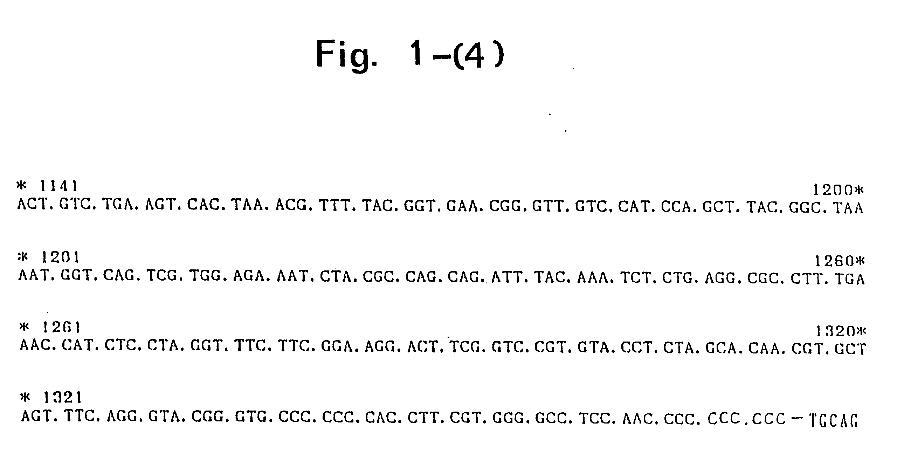

A DNA which contains the segment of the base sequence of the coat protein of the cucumber mosaic virus strain Y shown in Fig. 1 which begins at position 418 and ends at position 1071.

The DNA mentioned above which comprises the segment of the base sequence shown in Fig. 1 which begins at position 418 and ends at position 1071, with at least one codon selected from the group consisting of TAA, TAG and TGA at the 3' end.

A DNA coding for a polypeptide corresponding to the segment of the amino acid sequence shown in Fig. 1 which begins with the 1st amino acid and ends in the 218th amino acid or for a polypeptide derived therefrom by substitution, insertion, addition or deletion of one or more amino acid residues.

A vector DNA having intergrated the above DNA coding for the CP of CMV-Y being inserted therein.

A transformant like a bacterium, a plant cell or a plant as transformed with a vector DNA having the above DNA coding for the CP of CMV-Y inserted therein.

The invention can be accomplished in the manner mentioned hereinbelow.

(Extraction of RNA3 from CMV-Y and purification thereof)

For extracting RNA from CMV-Y, any of known methods for RNA extraction, for example the guanidine method, hot phenol method or SDS-phenol method, can be used. The strain CMV-Y can be obtained from the National Institute of Agrobiological Resources, Ministry of Agriculture, Forestry and Fishery, the Faculty of Agriculture, Kyoto University, or the Faculty of Agriculture, Iwate University, for instance. It may also be used as the raw material after purification in the conventional manner and storage (generally at -90°C to -60°C) in the form of a suspension in 10 mM phosphate buffer (pH 8.0) containing 1 mM ethylenediaminetetraacetic acid (EDTA), for instance.

RNA3 of CMV-Y can be isolated from the extracted CMV-Y RNA by any of known methods of fractionation, such as the method reported by Maniatis et al. [Molecular Cloning: A Laboratory Manual, Cold Spring Harbor Laboratory, N.Y. (1982)], low-melting (generally 45-60°C) agarose electrophoresis, column chromatography using an RDP minicolumn (commercial product of Bio-Rad Laboratories in U.S.A.), electroelution, or a modification of any of such methods. The RNA3 fraction can be detected using, as an indicator, the fact that RNA3 is the third in length among the four RNAs of CMV-Y.

(Synthesis of cDNA from RNA3)

From the solution of the isolated RNA3 of CMV-Y as obtained by the fractionation method, RNA3 can be obtained in a powder form by, for example, precipitaion with ethanol. This RNA3 is dissolved in 50 mM Tris-HCl buffer (pH 8.0) [containing 10 mM $MgCl_2$, 2.5 mM $MnCl_2$, 250 mM NaCl, 1 mM dithiothreitol (DTT), 0.05% (w/v) bovine serum albumin and 0.1 mM [$^3$H]adenosine triphosphate (ATP)] or the like and, after addition of a poly(A) sequence (preferably consisting of 10 to 20 A's) at the 3' end of RNA3 by using poly(A) polymerase, a template which is the product of addition of poly(A) to RNA3 at the 3' end thereof can be obtained in a powder form in the conventional manner, for example by precipitation with ethanol following deproteinization by extraction with phenol. This powder template is dissolved in 50 mM Tris-HCl buffer (pH 7.9) [containing 50 mM KCl, 10 mM $MgCl_2$, 3 mM DTT, 0.1% (w/v) Nonidet P-40 (Sigma), 20 μg/ml (dT)$_{12-18}$, 5 mM dATP, 5 mM dCTP, 5 mM dGTP, 5 mM dTTP, and 0.5 mM [$^3$H]deoxythymidine triphosphate (dTTP)], for instance, and subjected to the action of reverse transcriptase, for example one derived from Avian Myeloblastosis Virus or Rous associated virus (e.g. RAV-2 which is a commercial product of TAKARA SHUZO Co., Ltd. in Japan), with oligo(dT)$_{12-18}$ used as a primer, followed, if necessary, by alkalinization; a single-stranded cDNA can thus be synthesized. More specifically, from RNA3 of CMV-Y, there can be synthesized a single-stranded cDNA and then the corresponding double-stranded cDNA by suitably using, for example, the method reported by Okayama and Berg, the method reported by Sippel, the method reported by Gubler and Hoffman, the S1 method, the method reported by Land, or a modification of any of such methods.

(Cloning of cDNA)

The double-stranded cDNA is introduced, for example, into an Escherichia coli plasmid (e.g. pUC9) by the deoxyguanosine-deoxycytosine (dG-dC) method, for instance. The resultant plasmid can be used to transform Escherichia coli and so forth by the method reported by Mandel and Higa, for instance. The transformants, for example Escherichia coli transformants, are cultivated in an ampicillin-containing medium. From among the ampicillin-resistant strains thus grown, a strain carrying the longest cDNA (preferably having a base sequence of about 320 bp or more) is selected by preparing a plasmid, for example by the method reported by Maniatis et al. which comprises addition of a bacteriolytic enzyme (e.g. lysozyme), alkaline extraction and centrifugation (e.g. by the CsCl-ethidium bromide equilibrium density gradient method), for instance, and subjecting the plasmid to electrophoresis, among others. The strain thus selected is cultured in a liquid medium and, then, a plasmid (pCY3A27) in which the desired cDNA has been incorporated can be obtained by the method reported by Maniatis, for instance.

(Confirmation of the fact that the cDNA can serve as a probe for RNA3 of CMV-Y)

The plasmid pCY3A27 contains a cDNA which can be cleaved into two fragments (about 500 bp and about 300 bp) when treated with the restriction enzyme HindIII. These two fragments are subcloned and named pCY3A27HL and pCY3A27HS, respectively. pCY3A27HL can hybridize with RNA3 and RNA4 whereas pCY3A27HS can hybridize with RNA1, RNA2, RNA3 and RNA4. Therefore, pCY3A27HL can be used as a probe for RNA3.

(Cloning of the cDNA coding for the whole range of CP of CMV-Y)

Meanwhile, it is known that, in the Bromovirus, Ilarvirus, Alfalfa mosaic virus and Cucumovirus including CMV, the 3'-terminal 140-320 bases of the segmental genome RNAs (RNA1, RNA2, RNA3, RNA4) show a high degree of homology [Viral Genome Structure in the Viruses, vol. 1, 57 (1985)].

In view of the above facts, it is possible to synthesize a single-stranded cDNA using, as a primer, an oligonucleotide (5'-TGGTCTCCTT-3') complementary to the 3'-terminal ten bases (5'-AAGGAGACCA-3') of the RNA of the strain Q of CMV and, as template, the above-mentioned purified RNA3 of CMV-Y, together with reverse transcriptase. Subsequently, double-stranded cDNA synthesis, insertion into the Escherichia coli plasmid pUC9 and transformation of Escherichia coli with the resultant plasmid can be performed in the above-mentioned manner. Then, from among the transformants obtained, a clone capable of hybridizing with pCY3A27HL is obtained by colony hybridization using pCY3A27HL as the probe. A plasmid is prepared from this clone by the above-mentioned method reported by Maniatis, for instance. The size of the cDNA incorporated is measured, for example by electrophoresis following digestion of the plasmid obtained with the restriction enzyme PstI. The clone carrying a cDNA of about 1.4 kbp is cultured in a liquid medium and a plasmid is prepared as mentioned above by the method reported by Maniatis, for instance. The desired cDNA can be obtained by digesting the plasmid obtained with the restriction enzyme PstI. The base sequence of this cDNA can be determined, for example, by the method reported by Sangar et al. [Proceedings of the National Academy of Sciences of the United States of America, 74, 5463 (1977)].

The base sequence thus determined (Fig. 1) was identified as the base sequence for the CP of CMV-Y as a result of comparison, in respect of homology, with the partial amino acid sequence of the CP of CMV-Y as already described [Journal of Biochemistry, 97, 161 (1985)], with the 5'-terminal 106 bases of RNA4 of CMV-Y and with the CP of CMV-Q.

(Breeding of regenerated plants resistant to CMV)

The range of hosts to CMV is very wide, and CMV inflicts serious damage on crops such as tobacco, tomato and cucumber. Therefore, the cDNA obtained as described above which is a gene coding for the CP of CMV-Y is introduced into crop cells, for example tobacco cells, by a per se known method of gene introduction, for example the Agrobacterium method, electroporation, polyethylene glycol method, high pH-high calcium method, liposome method, microinjection method, or a modification of any of such methods, or a combination thereof, making use of the Ti plasmid, cauliflower mosaic virus "CaMV" or RNA virus which is known as a plant vector, or a vector expressible in plant cells which can be constructed by using the genetic engineering technology (e.g. one prepared by transforming promoter and terminator derived from CaMV into plasmid vector such as pUC9 and pUC19 derived from E. coli). Transformants are selected by using drug resistance as a marker. Drugs usable in this case are, for example, kanamycin, ampicillin, hygromycin and chloromycetin. The transformants obtained are cultured in an appropriate medium for callus formation, callus growth and, as necessary, adventitious embryo differentiation or organogenesis. They are then transferred to a medium for plant regeneration with a plant hormone added thereto and are caused to form seedlings, which are further caused to regenerate plants. In this way, virus-resistant crop plants can be produced.

In the case of tobacco, for instance, callus formation and callus growth can be realized by cultivating a transformant (protoplast) in a known medium or a modification thereof, for example MS medium [Murashige & Skoog medium; Physiologia Plantarum, 15, 473-479 (1962)], B5 medium [Experimental Cell Research, 50, 151-158 (1968)], Nagata & Takebe medium [Planta, 99, 12 (1971)], Kao & Michayluk (8p) medium [Planta, 126, 105 (1975)], Shahin TM-2 medium [Theoretical and Applied Genetics, 69, 235 (1985)], or a modification of any of such media.

More specifically, in the case of regeneration of tobacco plants, seedling formation is carried out using, as a regeneration medium, a medium obtained by modifying one of the above-mentioned media with respect to the hormone composition, for example such that an auxin amounts to not more than 5mg/ℓ (preferably 0.1 to 5 mg/ℓ) and a cytokinin to not more than 10/mg/ℓ (preferably 0.3 to 10 mg/ℓ). Examples of such auxin are 2,4-D, 2,3-dichlorophenylacetic acid, indole-3-acetic acid, indole-3-butyric acid, para-chlorophenoxyacetic acid, 2,4,5-trichlorophenoxyacetic acid and 1-naphthaleneacetamide. The cytokinin includes, among others, adenine type cytokinins and urea cytokinins. Usable as the adenine type cytokinins are, for example, 6-benzyladenine, 6-benzyladenosine, 2-isopentenyladenine, kinetin, zeatin, dihydrozeatin, zeatin riboside, 4-benzylaminobenzimidazole and 4-benzylaminopyrazolopyrimidine whereas diphenylurea, N-(2-methyl-4-pyridyl)-N'-phenylurea, N-(2,6-dichloro-4-pyridyl)-N'-phenylurea, N-(2,6-dichloro-4-pyridyl)-N'-phenylurea and N-(2-chloro-4-pyridyl)-N'-phenylurea, for instance, are used as the urea-type cytokinins.

The seedlings obtained are then transferred to a hormone-free medium and caused to root and grow to plant bodies. The plants thus obtained show resistance to viruses and are useful as materials for breeding virus-resistant crop plants.

In plant cells transformed with the DNA according to the invention, the CP of CMV-Y, which cannot be synthesized at all in natural plant cells in themselves, can be produced. The resitance to CMV infection as provided by said DNA can be shown not only in plant cells themselves but also in redifferentiated plant bodies.

In the present specification and drawings, the abbreviations used for indicating bases, amino acids and so forth are those abbreviations that are recommended by the IUPAC-IUB Commission on Biochemical Nomenclature or are conventionally used in the field of art which is concerned. Examples of such abbreviations are given below. When optical isomerism is possible for an amino acid, the amino acid, unless otherwise specified, is to be construed as being in the L form.

| | |
|---|---|
| DNA | Deoxyribonucleic acid |
| RNA | Ribunucleic acid |
| A | Adenine |
| T | Thymine |
| G | Guanine |
| C | Cytosine |
| dATP | Deoxyadenosine triphosphate |
| dTTP | Deoxythymidine triphosphate |
| dGTP | Deoxyguanosine triphosphate |
| dCTP | Deoxycytidine triphosphate |
| ATP | Adenosine triphosphate |
| EDTA | Ethylenediaminetetraacetic acid |
| SDS | Sodium dodecyl sulfate |
| DTT | Dithiothreitol |
| Gly | Glycine |
| Ala | Alanine |
| Val | Valine |
| Leu | Leucine |
| Ile | Isoleucine |
| Ser | Serine |
| Thr | Threonine |
| Cys | Cysteine |
| Met | Methionine |
| Glu | Glutamic acid |
| Asp | Aspartic acid |
| Lys | Lysine |
| Arg | Arginine |
| His | Histidine |
| Phe | Phenylalanine |
| Tyr | Tyrosine |
| Trp | Tryptophan |
| Pro | Proline |
| Asn | Asparagine |
| Gln | Glutamine |
| M | Molar concentration (mol/liter) |

The following examples are further illustrative of the present invention but are by no means limitative thereof.

Example 1 Isolation of RNA contained in CMV (strain Y)

Tobacco plants were inoculated with CMV-Y allotted from Iwate University. After growing at 25°C for 4 to 10 days, infected leaves (50 g) were harvested. The infected leaves were frozen (-20°C) and homogenized in 50 ml of 0.5 M sodium citrate buffer (pH 6.5) [containing 0.1% (w/v) thioglycolic acid and 10 mM EDTA and 50 ml of chloroform. The emulsion obtained was centrifuged at 4,600 × g for 15 minutes. The supernatant containing CMV-Y was centrifuged at 78,000 × g for 2.5 hours, and the sediment was suspended in 2 ml of 10 mM phosphate buffer (pH 7.0) [containing 2 mM EDTA and 1% (w/v) Triton X-100 ( a commercial product of Wako Pure Chemical Industries in Japan)]. The suspension was allowed to stand overnight at 4°C and then centrifuged at 8,700 × g for 15 minutes. The supernatant thus obtained was further centrifuged at 78,000 × g for 2.5 hours, and the sediment (containing virus particles) was suspended in 10 ml of 10 mM phosphate buffer (pH 7.0) [containing 2 mM EDTA]. Further two repetitions of the fractional centrifugation cycle (8,700 × g 15 minutes, and then 105,000 × g 2 hours) gave purified CMV-Y particles (28 mg). The purified CMV-Y particles thus obtained were suspended in 28 ml of 10 mM phosphate buffer (pH 7.0) [containing 2 mM EDTA] and stored at -80°C. For RNA isolation, the particles so stored were collected by centrifugation at 105,000 × g for 60 minutes and dried under reduced pressure. This dried sample (about 4 mg) was suspended in 1 ml of 20 mM Tris-HCl buffer (pH 8.0) [containing 1 mM EDTA]. Thereto was added 1 ml of 50 mM Tris-HCl buffer for RNA extraction (pH 7.2) [containing 1 mM EDTA, 50 mM NaCl, 0.1% (w/v) bentonite and 1% (w/v) sodium lauryl sulfate]. To the resultant mixture was further added 2 ml of water-saturated phenol, and the whole mixture was shaken vigorously at 25°C for 15 minutes. Thereafter, the aqueous layer was recovered by centrifugation at 25°C and 3,500 rpm for 5 minutes. To the aqueous layer obtained, there was again added 1 ml of water-saturated phenol, the mixture was shaken vigorously at 25°C for 5 minutes and then centrifugation was carried out in the same manner as just mentioned above. After further two repetitions of the above procedure, 1 ml of diethyl ether was added and the mixture was shaken thoroughly for removing the phenol contained in the aqueous layer. To the aqueous layer then separated, there were added 1/10 volume of 3 M sodium acetate (pH 5.2) and 2 volumes of ethanol (EtOH). The mixture was allowed to stand overnight at -20°C and then centrifuged at 3,500 rpm and 0°C for 15 minutes to give RNA (0.5 mg) as a sediment. This sediment was washed thoroughly with 4 ml of 70% aqueous EtOH solution and then dried under reduced pressure. To the dried sediment, there was added 200 μl of 20 mM Tris-HCl buffer (pH 8.0) [containing 1 mM EDTA], and the resultant solution was stored at -20°C.

Example 2 Screening for cDNA capable of serving as a probe for RNA3 and RNA4

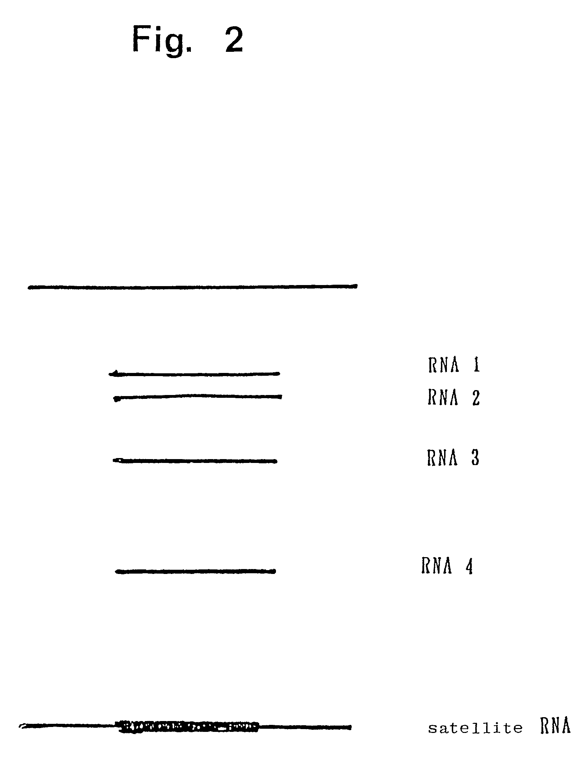

(1) The RNA solution obtained in Example 1 was subjected to electrophoresis in 89 mM Tris-HCl buffer (pH 8.3) [containing 89 mM boric acid and 2 mM EDTA] at 100 V for 3.5 hours using a slab gel (with ethidium bromide added in advance: so as to give a concentration of 0.2 μg/ml)of a 1% (w/v) low-melting agarose (#5517UA which is a commercial product of Bethesda Research Laboratories Life Technologies in U.S.A.). After electrophoresis, five bands ascribable to RNA1 to RNA5 of CMV-Y were observed under UV irradiation (the electrophoretogram obtained being shown in Fig. 2). Based on the mobility data, the third band from the top was considered to be the RNA3 fraction. The RNA3-containing gel (volume: 2 ml) was excised, 10 ml of 10 mM Tris-HCl buffer (pH 8.0) [containing 1 mM EDTA] was added, and the resultant mixture was warmed at 65° C for 5 minutes for dissolution of the gel and then cooled to 25° C. Thereto was added 12 ml of water-saturated phenol and, after thorough mixing, the resultant mixture was centrifuged at 3,000 × g for 5 minutes. To the aqueous layer obtained, there was added again 12 ml of water-saturated phenol. After vigorous shaking, the mixture was centrifuged at 3,000 × g for 5 minutes, 12 ml of a 1:1 mixture of phenol and chloroform was added to the aqueous layer and, after vigorous shaking, the mixture was centrifuges at 3,000 × g for 3 minutes. To the obtained aqueous layer, 12 ml of chloroform was added, and after vigorous shaking, the mixture was centrifuged at 3,000 × g for 3 minutes. The aqueous layer thus obtained was extracted three times with diethyl ether to thereby remove the remaining phenol and chloroform. To the aqueous layer, there were added 1/10 volume of 3 M sodium acetate (pH 5.2)and 2.5 volumes of ethanol, and the resultant mixture was allowed to stand overnight at -20° C. Then, centrifugation was carried out at 3,000 × g for 15 minutes, the sediment obtained was washed with 10 ml of 70% aqueous ethanol and centrifugation was carried out again at 3,000 × g for 10 minutes. The sediment obtained was dried under reduced pressure and dissolved in 20 μl of sterilized water. The nucleic acid content was determined using the extinction coefficient

$$E\frac{0.1\%}{260 \text{ nm}} = 22,$$

and the yield of purified RNA3 was calculated to be 20 μg.

(2) To 10 μl of the aqueous solution of RNA3 (10 μg), there were added 10 μg of ATP and 12 units of poly(A) polymerase (E. coli poly(A) polymerase #8032SA/SB which is a commercial product of Bethesda Research Laboratories Life Technologies) and the poly(A) addition reaction was carried out in 100 μl of 50 mM Tris-HCl buffer (pH 8.0) [containing 10 mM MgCl₂, 2.5 mM MnCl₂, 250 mM NaCl, 0.5 mg/ml bovine serum albumin (#27-8915-01 which is a commercial product of P-L Biochemicals, Inc, in U.S.A.), 1mM dithiothreitol and 0.2 unit/μl ribonuclease (RNase) inhibitor (#121800 which is a commercial product of Promega Biotec in U.S.A.) at 37° C for 90 minutes. The reaction mixture was extracted with two portions of a phenol-chloroform (1:1) mixture, followed by addition of 100 μl of 4 M ammonium acetate and 400 μℓ of ethanol. The resultant mixture was allowed to stand at -70° C for 1 hour. Centrifugation was carried out at 14,000 rpm for 10 minutes, and the sediment obtained was washed with 500 μl of 70% (w/v) aqueous ethanol. After centrifugation at 14,000 rpm for 5 minutes, the sediment was dried under reduced pressure to give 10 μg of powdery RNA3 with poly(A) added thereto.

(3) To a solution of 10 μg of the powdery RNA3 obtained as described above under (2) in 10 μℓ of sterilized water, there was added an equal volume of 4 M ammonium acetate, followed by further addition of 2 volumes of ethanol. The resultant mixture was allowed to stand at -70° C for 30 minutes, followed by centrifugation at 15,000 rpm for 15 minutes. The sediment was washed with 70% (w/v) aqueous ethanol. After centrifugation under the same conditions, the sediment was dried under reduced pressure. To the sediment, there were added, in sequence, 5 μl of a nucleotide solution [for the first strand; 5 mM dATP (#27-5500 which is a commercial product of P-L Biochemicals, Inc. in U.S.A.), 5 mM dCTP (#27-5600 which is a commercial product of P-L Biochemicals, Inc.), 5 mM dGTP (#27-5700 which is a commercial product of P-L Biochemicals, Inc.) and 5 mM dTTP (#27-5800 which is a commercial product of P-L Biochemicals, Inc.)], 2 μl of 500 mM Tris-HCl buffer for the first strand (pH 8.3) [containing 100 mM MgCl₂ and 100 mM dithiothreitol], 2 μl of oligo-(dT)₁₂₋₁₈ (1 μg/μl)(#27-7858 which is a commercial product of P-L Biochemicals, Inc.), 2 μl of 40 mM sodium pyrophosphate and 20 μCi of [α-³²p]dCTP (3,000 Ci/mmol, a commercial product of Amersham Corporation in England), and the mixture was heated at 65° C for 5 minutes and then cooled to 25° C. To this solution, there were added 1.0 μl of RNase inhibitor (40 units/μl; #121800 which is a commercial product of Promega Biotec) and 6 μl of reverse tran-

scriptase (10 units/μl; #27-0921 which is a commercial product of P-L Biochemicals, Inc.), and the reaction was carried out at 43° C for 90 minutes. Thereto were added 2 μl of 0.25 M EDTA and 1 μl of 10% (w/v) sodium lauryl sulfate and, after mingling, the reaction was discontinued. A phenol-chroloform mixture (20 μl) [prepared by saturating a mixture of 250 ml of phenol, 240 ml of chloroform, 10 ml of isoamyl alcohol, 0.5 g of 8-quinolinol and 1 ml of 2-mercaptoethanol with 1 M Tris (pH not adjusted)] was added, the mixture was shaken vigorously and then centrifuged at 14,000 rpm for 2 minutes, 20 μl of 4 M ammonium acetate and 80 μl of ethanol were added to the supernatant, and the whole mixture was allowed to stand on dry ice for 15 minutes. The contents were thawed by warming and gentle shaking and, immediately thereafter, centrifuged at 14,000 rpm for 10 minutes. The sediment was dissolved in 10 μl of 10 mM Tris-HCl buffer (pH 8.0) [containing 1 mM EDTA]. Then, 10 μl of 4 M ammonium acetate and 40 μl of ethanol were added, and the mixture was again centrifuged at 14,000 rpm for 10 minutes. The sediment was washed with 50 μl of 70% aqueous ethanol (-20° C) and dried under reduced pressure. Then, 5 μl of 200 mM Tris-HCl buffer (pH 7.5) for the second strand [containing 50 mM MgCl₂, 100 mM (NH₄)₂SO₄ and 1 M KCl] and 25 μl of H₂O were added, and the thus-obtained sediment solution was stored at -70° C.

(4) To the 30-μl solution of the single-stranded cDNA (RNA/cDNA hybrid) as obtained as described above under (3), there were added, in sequence 41.5 μl of H₂O, 0.5 μl of the same Tris-HCl buffer for the second strand as mentioned above, 10 μl of a nucleotide solution [for the second strand; dATP, dCTP, dGTP and dTTP each 400 μM], 1.5 μl of 10 mM β-nicotinamide adenine dinucleotide (#632 which is a commercial product of Sigma Chemical Company in U.S.A.), 0.5 μl of deoxyribonuclease (DNase)-free bovine serum albumin (10 mg/ml;#27-8915 which is a commercial product of P-L Biochemicals, Inc.) and 50 μCi of [α-³²p]dCTP (3,000 Ci/mmol), and the mixture was maintained on ice. After further addition of 2 μl of ribonuclease H (0.5 unit/μl; #8021SA/SB which is a commercial product of Bethesda Research Laboratories Life Technologies) and 4.5 μl of DNA polymerase I (2.55 units/μl; P-L Biochemicals' #27-0926 which is a commercial product of P-L Biochemicals, Inc.), the reaction was carried out at 12° C for 2 hours and then at 22° C for 1 hour and, thereafter, discontinued by addition of 8 μl of 0.25 M EDTA. There was further added 100 μl of a

phenol-chloroform (1:1) mixture, the mixture was shaken vigorously and centrifuged at 14,000 rpm for 2 minutes, 100 μl of a phenol-chloroform (1:1) mixture was added to the supernatant, the mixture was centrifuged at 14,000 rpm for 2 minutes, 100 μl of 4 M ammonium acetate and 400 μl of ethanol were added to the supernatant, and the resultant mixture was allowed to stand on dry ice for 15 minutes, followed by centrifugation at 14,000 rpm for 10 minutes. The sediment was dissolved in 50 μl of 10 mM Tris-HCl buffer (pH 7.3) [containing 1 mM EDTA], and precipitation was again caused by adding 50 μl of 4 M ammonium acetate and 200 μl of ethanol. The sediment was washed with 250 μl of 70% (w/v) aqueous ethanol and dried under reduced pressure.

(5) To the double-stranded cDNA sediment obtained as described above under (4), there were added 20 μl of 50 mM Tris-HCl buffer (pH 6.9) [containing 400 mM sodium cacodylate and 2 mM CoCl₂], 4 μl of 20 mM dCTP, 2 μl of DNase-free bovine serum albumin (10 mg/ml; #27-8915 which is a commercial product of P-L Biochemicals, Inc.) and 12 μl of H₂O and, after further addition of 2 μl of terminal deoxynucleotidyl transferase (5.1 units/μl; #27-0730 which is a commercial product of P-L Biochemicals, Inc.), the reaction was carried out at 37° C for 7 minutes, followed by addition of 1 μl of 0.25 M EDTA for discontinuation of the reaction.

(6) To the reaction mixture containing the ds-cDNA (double strand-cDNA) with a dC tail as obtained as described above under (5), there were added 1 μl of 3'-oligo(dC)-tailed pUC9 (100 ng/μℓ; #27-4942 which is a commercial product of P-L Biochemicals, Inc.), 10 μl of 100 mM Tris-HCl buffer (pH 7.5) [containing 1 M NaCl and 10 mM EDTA] and 84 μl of H₂O. The mixture was heated at 65° C for 3 minutes, then quickly cooled to 42° C, maintained at that temperature for 2 hours, gradually cooled overnight, and stored at 4° C.

(7) E. coli JM 109 was inoculated into a plate of LM medium [prepared by dissolving 10 g of Bacto-tryptone, 5 g of Bacto-yeast extract, 0.58 g of NaCl and 15 g of Bacto-agar and, after cooling to 50 to 60° C,adding 240 μl of 25 mg/ml ampicillin and 1.5 ml of 1 M MgSO₄•7H₂O (subjected to bacterial filtration in advance; to be added after autoclaving of the medium) per 150 ml] and stored at 4° C. Five colonies were transferred to 3 ml of LB medium [composed of 10 g of Bacto-tryptone, 5 g of Bacto-yeast extract and 10 g of NaCl] and cultured at 37° C for 16 hours. SOB medium (40 ml) [composed of 20 g of Bacto-tryptone, 5 g of Bacto-yeast extract, 0.58 g of NaCl, 0.19 g of KCl, 10 ml of 1 M MgCl₂•6H₂O (subjected to bacterial filtration in advance; to be added after autoclaving of the medium) and 10 ml of 1 M MgCl₂•7H₂O (subjected to bacterial filtration in advance; to be added after autoclaving of the medium)] was placed in a sterilized 300-ml vessel. A 0.5 ml portion of the suspension of JM 109 as obtained by cultivation in LB medium was added to the vessel, and cultivation was carried out at 37°C and 130 revolutions per minute for 1.5 hours. After ice cooling for 10 to 15 minutes, the culture was centrifuged at 2,500 rpm and 4°C for 12 minutes. The cells thus collected were suspended in 3 ml of a buffer [10 mM CH₃COOK, 100 mM KCl, 45 mM MnCl₂•4H₂O, 10 mM CaCl₂•2H₂O, 3 mM [Co-(NH₄)₆]Cl₃ and 10% (v/v) glycerol (re-distilled glycerol produced by Bethesda Research Laboratories); adjusted to pH 6.4 with HCl] with gentle shaking, followed by further addition of 10 ml of the same buffer. After ice cooling for 10 to 15 minutes, the cells were collected by centrifugation at 2,500 rpm and 4°C for 10 minutes, and suspended in 3.2 ml of the same buffer with gentle shaking. To the cell suspension was added 115 μl of dimethyl sulfoxide (DMSO), and the mixture was lightly shaken and ice-cooled for 5 minutes. A further 115 μl portion of DMSO was added, and the mixture was lightly shaken, ice-cooled for 5 minutes and distributed, in 200 μl portions, into polypropylene culture tubes (#06480 which is a commercial product of Nissui Pharmaceutical Company Co., Ltd. in Japan). The tubes were put in liquid nitrogen and the contents thus frozen were stored at -70°C for 3 hours.

(8) The cells obtained as described above under (7) and stored at -70°C were maintained on ice for 15 minutes. Thereto was added 10 μl of the annealing reaction mixture obtained as described above under (6). The resultant mixture was shaken and then again allowed to stand on ice for 30 minutes. After heating at 42°C for 45 seconds and allowing to stand on ice for 2 minutes, the mixture was supplemented with 800 μl of SOC medium [prepared by adding 10 ml of 2 M glucose to 1 liter of SOB medium], and cultivation was conducted at 37°C and 120 revolutions per minute for 1 hour. A 200 μl portion of the culture obtained was inoculated onto an LMAX plate [prepared by layering, on LM medium, 3 ml of a medium obtained by dissolving 10 g of Bacto-tryptone, 5 g of Bacto-yeast extract, 0.58 g of NaCl and 15 g of Bacto-agar, cooling to 50 to 60°C and adding 12 μl of 1 M MgSO₄•7H₂O (subjected to bacterial filtration in advance; to be added after autoclaving of the medium), 60 μl of 25 mg/ml ampicillin, 500

μl of 2% (w/v) 5-bromo-4-chloro-3-indolyl-β-D-galactopyranoside in dimethylformamide and 200 μl of 100 mM isopropylthiogalactoside, per 30 ml]. The remaining 800 μl portion was centrifuged at 2,500 rpm for 10 minutes, and the cells collected were re-suspended in 200 μl of SOC medium and inoculated onto another LMAX plate. Each LMAX plate was air-dried for 10-20 minutes and incubated upside down at 37°C for 14 hours.

(9) The colonies obtained as described above under (8) were transferred to 3 ml of LB medium supplemented with 50 μg/ml of ampicillin and shake-cultured overnight at 37°C. A 1.5 ml portion of the culture was centrifuged at 6,000 rpm for 2 minutes. The supernatant was removed, and the sediment was suspended in 80 μl of Tris-HCl buffer (pH 8.0) [containing 62.5 mM glucose and 12.5 mM EDTA], 20 μl of 20 mg/ml aqueous lysozyme solution was added, and the reaction was carried out at 25°C for 5 minutes. 0.2 N NaOH/1% SDS (200 μl) (prepared just prior to use) was added and, after turning upside down 2 or 3 times for thorough mixing, the mixture was cooled on ice for 5 minutes. Further, 200 μl of ice-cooled 3 M potassium acetate (pH 4.8) was added, and the mixture was shaken vigorously for about 10 seconds, then ice-cooled for 5 minutes and centrifuged at 14,000 rpm for 5 minutes. To the supernatant was added 500 μl of a phenol-chloroform (1:1) mixture, followed by vigorous shaking. The mixture obtained was centrifuged at 14,000 rpm for 2 minutes, 800 μl of ethanol was added to the supernatant. The mixture was shaken vigorously, then allowed to stand at 25°C for 2 minutes and centrifuged at 14,000 rpm for 5 minutes. The supernatant was removed, and 1 ml of 70% (w/v) aqueous ethanol was added to the sediment. The mixture was shaken thoroughly for effecting washing of the sediment, and then centrifuged at 14,000 rpm for 5 minutes. The supernatant was removed, and the sediment was dried under reduced pressure and dissolved in 50 μl of 10 mM Tris-HCl buffer (pH 8.0) [containing 20 μg/ml RNase (ribonuclease A) and 1 mM EDTA] by adding the buffer to the sediment. A 10 μl portion of the solution was treated with the restriction enzyme PstI, followed by electrophoresis. After electrophoresis, staining was carried out with 0.5 μg/ml ethidium bromide and the chain length of the cDNA was calculated. As a result, 1 μg of a plasmid containing the longest cDNA (plasmid pCY3A27) was obtained.

(10) The plasmid pCY3A27 obtained as described above under (9) was digested with the restriction enzyme HindIII in 10 mM Tris-HCl buffer (pH 8.0) [containing 60 mM NaCl, 7 mM MgCl₂, 7 mM 2-mercaptoethanol and 0.1 mg/ml bovine serum albumin at 37°C for 3 hours, whereby the insert cDNA was divided into two fragments (about 500 bp and about 300 bp). These two fragments were cloned and named pCY3A27HL (about 500 bp) and pCY3A27HS (about 300 bp), respectively. The RNA (2.5 $\mu$g) solution obtained in the same manner as in Example 1 was heated in a denaturing solution (pH 7.0) [composed of 50% (v/v) formamide, 2.2 M formaldehyde, 20 mM sodium 3-(N-morpholino)propanesulfonate (MOPS-Na), 5 mM sodium acetate and 1 mM EDTA] at 55°C for 15 minutes and then cooled on ice. After addition of 1/10 volume of a loading buffer [composed of 50% (v/v) glycerol, 1 mM EDTA, 0.4% bromophenol blue and 0.4% (w/v) xylene cyanol], the resultant mixture was electrophoresed in 1.5% (w/v) agarose gel (pH 7.0) [containing 2.2 M formaldehyde, 20 mM MOPS-Na, 5 mM sodium acetate and 1 mM EDTA] at 50 V for 18 hours. Thereafter, the RNAs were transferred to a nitrocellulose membrane (BA85 which is a commercial product of Schleicher & Schuell GmbH in West Germany) and hybridized with the plasmid DNAs in which pCY3A27HL and pCY3A27HS had been cloned respectively and which had been labeled using a nick translation kit (#5000 which is a commercial product of Amersham Corporation). As a result, pCY3A27HL hybridized with RNA3 and RNA4 and pCY3A27HS hybridized with RNA1, RNA2, RNA3 and RNA4. It was thus found that pCY3A27HL can serve as a probe for RNA3 and RNA4.

Example 3 Cloning of cDNA coding for the CP gene

(1) Cloning was carried out in the same manner as in Example 2 (1) to (9) except that 2 $\mu$l of oligo(dT)₁₂₋₁₈ (1 $\mu$g/$\mu$l) was replaced with 2 $\mu$l of the oligonucleotide 5′ TGGTCTCCTT 3′ - (produced by Takara Shuzo Co., Ltd. in Japan) in the same concentration (1 $\mu$g/$\mu$l) and that the first strand synthesis was conducted at 32°C for 60 minutes and further at 43°C for 90 minutes.
(2) Colony hybridization was carried out with the cDNAs obtained as described above under (1) using pCY3A27HL obtained in Example 2 (10) as a probe. As a result, several positive clones were obtained. The plasmid DNA was extracted from each of these clones and electrophoresed in the same manner as in Example 2 (1) after digestion of the plasmid with Pst I (produced by Takara Shuzo Co., Ltd.), whereby a clone (E. coli JM109/pCYCI) harboring a plasmid(pCYCI)

affording DNA fragment about 1,400 bp was obtained. The strain harboring this clone was shake-cultured in 5 ml of LB medium [containing 50 $\mu$g/ml ampicillin] at 37°C for 15 hours. A 0.1 ml portion of the culture obtained was shake-cultured in 25 ml of LB medium [containing 50 $\mu$g/ml ampicillin] at 37°C until the turbidity amounted to 100 Klett units (wavelength: 580 nm). The whole amount of the culture obtained was added to 500 ml of LB medium [containing 50 $\mu$g/ml ampicillin], and shake culture was carried out at 37°C for 2.5 hours. Then, chloramphenicol was added in a concentration of 170 $\mu$g/ml, and shake culture was continued at 37°C for 15 hours. Cells were harvested by centrifugation at 4°C and 5,000 rpm for 10 minutes. To the cells was added 10 ml of 25 mM Tris-HCl buffer (pH 8.0) [containing 5 mg/ml egg-white lysozyme, 50 mM glucose and 10 mM EDTA], the mixture was allowed to stand at 4°C for 30 minutes, 20 ml of 0.2 N NaOH [containing 1% (w/v) SDS] was then added, and the resultant mixture was allowed to stand on ice for 10 minutes. Then, 15 ml of 5 M potassium acetate (pH 4.8) was added and, after allowing to stand on ice for 10 minutes, the mixture was centrifuged at 4°C and 8,000 rpm for 10 minutes. To the supernatant was added 2.5 volumes of ethanol, and the mixture was allowed to stand at 20°C for 15 minutes and then centrifuged at 20°C and 8,000 rpm for 10 minutes. The sediment obtained was dissolved in 10 ml of 10 mM Tris-HCl buffer (pH 8.0) [containing 1 mM EDTA], followed by addition of 0.1 ml of a solution of 30 mg of ethidium bromide in 1 ml of dimethyl sulfoxide and 11 g of CsCl. The mixture thus obtained was centrifuged at 20°C and 50,000 rpm for 15 hours. A plasmid fraction exhibiting fluorescence under UV irradiation was collected. An equal volume of n-butanol was added to this fraction and, after stirring, the aqueous layer which had resulted was separated. The same procedure was repeated two times more, and the aqueous layer finally obtained was dialyzed [dialyzing solution: 10 mM Tris-HCl buffer (containing 1 mM EDTA)] for removing CsCl. A 100 $\mu$l portion of the dialyzate was treated with 300 units of the restriction enzyme PstI at 37°C for 1.5 hours and then electrophoresed in 89 mM Tris-HCl buffer (pH 8.3) [containing 89 mM boric acid and 2 mM EDTA] at 100 V for 3.5 hours using a slab gel (the gel containing 0.2 $\mu$g/ml ethidium bromide) of a 1% (w/v) low-melting agarose (#5517UA which is a commercial product of Bethesda Research Laboratories Life Technologies). After electrophoresis, the band corresponding to 1.4 kbp was collected under UV irradiation, warmed at 65°C for 15 minutes for melting of agarose, and applied to an RDP mini-column (produced by Bio-Rad Laboratories) to give a 1.4 kbp cDNA fragment. The cDNA obtained showed the base sequence shown in Fig. 1. The transformant obtained in the above manner, namely Escherichia coli JM 109/pCYCI, has been deposited at the Institute for Fermentation, Osaka (IFO) since February 5, 1987 under the deposit number IFO-14572 and also at the Fermentation Research Institute (FRI), Agency of Industrial Science and Technology, Ministry of International Trade and Industry since February 21, 1987 under the deposit number FERM BP-1683.

### Example 4: Construction of pCAM35

(1) One $\mu$g of plasmid pUC9 was dissolved in MR buffer [containing 10 mM Tris-HCl buffer (pH 7.5), 10 mM MgCl$_2$, 1 mM dithiothreitol (DTT), 50 mM NaCl], and was digested with 10 units of HincII (produced by Takara Shuzo Co., Ltd. in Japan) and 10 units of PstI (produced by Takara Shuzo Co., Ltd.) at 37°C for 2 hours. From the reaction mixture, eight hundred ng of DNA fragment was recovered by ethanol precipitation as described in Example 2(3). Eight hundred ng of the DNA fragment was dissolved in TP buffer [containing 67 mM Tris-HCl buffer (pH 8.8), 6.7 mM MgCl$_2$, 16.6 mM (NH$_4$)$_2$SO$_4$, 10 mM 2-mercaptoethanol, 6.7 $\mu$M EDTA, 0.0167% bovine serum albumin (BSA)], and was reacted with 5 units of T4 DNA polymerase (produced by Takara Shuzo Co., Ltd.) and 5 $\mu$l of dNTP solution [containing 1 mM dATP, 1 mM dGTP, 1 mM dCTP, and 1 mM dTTP] at 37°C for 30 minutes to create blunt ends. After deproteinization of the reaction mixture with chloroform-phenol (1:1) mixture, the DNA fragment was recovered by ethanol precipitation as described in Example 2(1). The DNA fragment was circularized by self-ligation with the DNA ligation kit (produced by Takara Shuzo Co., Ltd., code: 6021). As described in Example 2(8), the circularized DNA was transformed into competent cells of E.coli DH5$\alpha$, which were prepared as described in Example 2(7). From each colony, plasmid was isolated as described in Example 2(9). After digestion of the plasmid with BamHI (produced by Takara Shuzo Co., Ltd.) at 37°C for 2 hours in HR buffer [containing 50 mM Tris-HCl buffer (pH 7.5), 10 mM MgCl$_2$, 1 mM DTT, 100 mM NaCl] and electrophoresis of the reaction mixture, the clone affording ca. 2.6 kbp DNA fragment was selected. The plasmid cloned as described above, was named pUC91. Then 50 $\mu$g of the plasmid pUC91 was obtained as described in Example 3(2).

(2) Ten $\mu$g of cauliflower mosaic virus (CaMV)-CM1841 was dissolved in MR buffer, and was digested with 20 units of EcoRI (produced by Takara Shuzo Co., Ltd.) at 37°C for 2 hours. The reaction mixture was subjected to electrophoresis on agarose gel as described in Example 3(2), and the two bands, one corresponding to 0.5 kbp DNA fragment and the other to 2.3 kbp DNA, were cut off. From each agarose gel piece, DNA fragment was eluted and purified by using Gene Crean (produced by Funakoshi Pharmaceutical Company Co. Ltd. in Japan). Each eluate was subjected to deproteinization with chloroform-phenol (1:1) mixture and ethanol precipitation, and 1 $\mu$g of 2,334 bp DNA fragmennt, named FRC, and 0.2 $\mu$g of 459 bp DNA fragment, named FRD, were obtained.

(3) One $\mu$g of plasmid vector pUC13 was dissolved in HR buffer, and was digested with 5 units of EcoRI at 37°C for 2 hours. To the reaction mixture, equal volume of 2 M Tris-HCl buffer pH 8.0 and 5 units of alkaline phosphatase (produced by Takara Shuzo Co., Ltd.: code 2021A) were added. After incubation at 65°C for 30 minutes, equal volume of phenol saturated with sterilized water was added. The mixture was shaken vigorously and centrifuged at 15,000 rpm and 4°C for 2 minutes. Subsequently equal volume of diethylether was added to the aqueous layer, and the mixture was shaken and centrifuged at 6,000 rpm. Six hundred ng of dephosphorylated open-circular DNA was obtained after ethanol precipitation of the aqueous layer.

One $\mu$g of the DNA fragment FRC obtained in (2) and 0.3 $\mu$g of thus obtained dephosphorylated open-circular plasmid vector pUC13 were ligated with the DNA ligation kit and cloned, and the resultant plasmid was named pCMEC2+. Ten $\mu$g of pCMEc2+ was dissolved in HR buffer, and was digested with 20 units of BglII (produced by Takara Shuzo Co., Ltd.) at 37°C for 2 hours. The reaction mixture was subjected to electrophoresis on agarose gel, and the band corresponding to the smaller DNA fragment was cut off. From the gel piece, 0.5 $\mu$g of the DNA fragment was eluted and purified. 0.5 $\mu$g of the DNA fragment was cloned into the phosphatase-treated BamHI site of pUC91 obtained in (1), and the resultant plasmid was named pCI.

(4) One $\mu$g of pCI obtained in (3) was dissolved in SS buffer [containing 10 mM Tris-HCl buffer (pH 8.0), 7 mM MgCl$_2$, 20 mM KCl, 7 mM 2-mercaptoethanol, 0.01% BSA], and was digested with 5 units of SmaI (produced by Takara Shuzo Co., Ltd.) and 10 units of HincII at 30°C for 2 hours. The reaction mixture was subjected to electrophoresis on agarose gel, and the band corresponding to the larger DNA fragment was cut off. From the gel piece, 0.5 μg of the DNA fragment was eluted and purified. The DNA fragment was circularized by self-ligation with the DNA ligation kit, and was cloned. The resultant plasmid was named pC2.

Five μg of pC2 was dissolved in HS buffer [containing 6 mM KCl, 10 mM Tris-HCl buffer pH 7.4, 10 mM MgCl₂, 1 mM DTT, 200 μg/ml BSA], and was digested with 20 units of HphI (produced by New England Biolabs Inc. in U.S.A.) at 37°C for 2 hours. The reaction mixture was subjected to electrophoresis on agarose gel, and the band (A) corresponding to ca. 0.8 kbp DNA fragment and the band (B) corresponding to ca. 1.3 kbp DNA fragment were cut off, respectively.

0.3 μg of 799 bp DNA fragment containing the promoter originated from CaMV was eluted and purified from the gel piece of (A). 0.3 μg of the DNA fragment was dissolved in HR buffer, and was digested with 5 units of EcoRI at 37°C for 2 hours, and then was recovered by ethanol precipitation. The recovered DNA fragment was cloned into the EcoRI-SmaI site of the plasmid vector pUC19. The resultant plasmid was named pC2P.

One μg of 1,338 bp DNA fragment containing the terminator was eluted and purified from the gel piece of (B). One μg of the DNA fragment was dissolved in MR buffer, and was digested with 5 units of HindIII (produced by Takara Shuzo Co., Ltd.) at 37°C for 2 hours, and the DNA fragment was recovered by ethanol precipitation. The recovered DNA fragment was cloned into the HindIII-SmaI site of the plasmid vector pUC19. The resultant plasmid was named pC2T.
(5) The DNA fragment FRD described in (2), was cloned into the phosphatase-treated EcoRI site of pUC19. The resultant plasmid was named pECO46. Five μg of pECO46 was dissolved in MR buffer and was digested with 10 units of PstI and 10 units of HindIII at 37°C for 2 hours. The reaction mixture was subjected to electrophoresis on agarose gel, and the band corresponding to ca. 0.25 kbp DNA fragment was cut off. From the agarose gel piece, 0.8 μg of DNA fragment containing polylinker site was eluted and purified. The DNA fragment was cloned into the PstI-HindIII site of pC2P obtained in (4). The resultant plasmid was named pC33.

Five μg of pC33 was dissolved in LR buffer [containing 10 mM Tris-HCl buffer pH (7.5), 10 mM MgCl₂, 1 mM DTT], and was digested with 20 units of KpnI (produced by Takara Shuzo Co., Ltd.) at 37°C for 2 hours, followed by the addition of equal volume of HR buffer, and the digestion reaction was continued with 20 units of EcoRI at 37°C for 2 hours. The reaction mixture was subjected to electrophoresis on agarose gel, and the band corresponding to the smaller DNA fragment was cut off. From the gel piece, 1.5 μg of DNA fragment which contained the promoter originated from CaMV and the polylinker site, was eluted and purified. The DNA fragment was cloned into the KpnI-EcoRI site of pC2T constructed in (4) to obtain a transformant, E. coli DH5α/pCAM35 (deposited at IFO since February 3, 1988 under the deposit No. IFO-14724 and also at FRI since February 10, 1988 under the deposit No. FERM BP-1727). The resultant plasmid was named pCAM35.

Example 5: Construction of pNR

Ten μg of the plasmid pNEO (produced by Pharmacia Fine Chemicals in Sweden, code: 27-4924-01) containing kanamycin resistance gene (NPT II) was dissolved in SS buffer as described above in Example 4(4), and was digested with 30 units of BglII and 30 units of SmaI at 30°C for 3 hours. The reaction mixture was subjected to electrophoresis on agarose gel and the band corresponding to ca. 1.0 kbp DNA fragment was cut off. From the gel piece, 2 μg of 1,003 bp DNA fragment containing NPT II gene was eluted and purified. The DNA fragment was cloned into the BamHI-SmaI site of pCAM35 constructed in Example 4(5). The resultant plasmid was named pNR.

Example 6: Construction of pCCP

(1) Twenty μg of the plasmid pCYCI constructed in Example 3(2) was dissolved in MR buffer described in Example 4(1), and was digested with 50 units of HaeII (produced by Takara Shuzo Co., Ltd.) at 37°C for 2 hours. The reaction mixture was subjected to electrophoresis on agarose gel, and the band corresponding to ca. 1,4 kbp DNA fragment was cut off. From the gel piece 5 μg of 1,461 bp DNA fragment was eluted and purified. The DNA fragment was dissolved in TP buffer described in Example 4(1), and was reacted with 5 units of T4 DNA polymerase and 5 μl of dNTP solution described in Example 4(1) at 37°C for 30 minutes to create blunt ends. After deproteinization as described in Example 4(1), 5 μg of the DNA fragment was recovered by ethanol precipitation. Five μg of the DNA fragment was dissolved in HR buffer described in Example 4(1), and was digested with 10 units of PstI at 37°C for 2 hours. The reaction mixture was subjected to electrophoresis on agarose gel, and the band corresponding to ca. 1.2 kbp DNA fragment was cut off. From the gel piece, 2 μg of 1,260 bp DNA fragment was eluted and purified. The DNA fragment was cloned, by using competent cells of E. coli JM109 which had been prepared as described in Example 2(7), into the PstI-SmaI site of the plasmid pCAM35 constructed in Example 4(5). The resultant plasmid was named pA1.

(2) Ten μg of pA1 obtained in (1) was dissolved in MR buffer, and was digested with 20 units of PstI and 30 units of BanII at 37°C for 2 hours. The reaction mixture was subjected to electrophoresis on agarose gel, and the band corresponding to the larger DNA fragment was cut off. From the gel piece, 5 μg of 4,260 bp DNA fragment was eluted and purified. Five μg of the DNA fragment was dissolved in TP buffer, and was treated with 5 units of T4 DNA polymerase and 5 μl of dNTP solution at 37°C for 30 minutes to create blunt ends. From the reaction mixture, 5 μg of DNA fragment was recovered by ethanol precipitation. Two μg of the DNA fragment was circularized by self-ligation as described in Example 4(4), and then cloned to obtain a transformant E. coli JM 109/pCCP#1 (deposited at IFO since February 3, 1988 under the deposit No. IFO-14723 and also at FRI since February 10, 1988 under the deposit No. FERM BP-1726 ). The resultant plasmid was named pCCP.

Example 7 Transformation of tobacco protoplasts by electroporation

(1) Isolation of Nicotiana tabacum L. c.v. Bright Yellow protoplasts:

The tobacco plants were grown under greenhouse condition. The fifth fully expanded leaves of 2-month old tobacco plants were immersed in 70% aqueous ethanol for 10 seconds and then in 1% sodium hypochloride for 10 minutes followed by washing with sterile water three times. Subsequently the lower epidermis was removed and the peeled leaflets were floated, with their lower surface downwards, in a filter sterilised enzyme mixture (pH 5.5) consisting of 1% Cellulase Onozuka R-10 (a commercial product of Yakult Co., Ltd. in Japan), 0.1% Macerozyme R-10 (a commercial product of Yakult Co., Ltd.) and 0.4 M mannitol. Digestion was completed after 4-hour incubation in the dark at 26°C. Isolated protoplasts were washed three times with K3 medium [Protein, Nucleic Acid and Enzyme, 30, 119 (1987)].

(2) Electroporation

Co-transformation [Bio/Technology, 4, 1093 (1986)] in the presence of pCCP and pNR by electroporation:

Tobacco protoplasts were suspended at the concentration of $5 \times 10^5$ protoplasts/ml in a poration medium (1 ml) [Plant Cell Reports, 5, 57 (1986)] mixed with 30 μg of pNR containing the kanamycin-resistance gene and 30 μg of pCCP containing the CMV coat protein gene. The suspension (300 μl) was layered in the space (2 mm) between electrodes of the fusion chamber (SSH-CO3 which is a commercial product of Shimadzu Corporation in Japan) at 0°C on the temperature control stage (SSH-T1 which is a commercial product of Shimadzu Corporation), and was subjected to an electrical pulse of 60 μ seconds duration at the field strength of 0.5-1.0 kV/cm by electrofusion apparatus (Somatic hybridizer, SSH-1 which is a commercial product of Shimadzu Corporation). After electroporation the protoplast samples were held at 0°C for 10 min. The protoplasts were resuspended in K3 medium and cultured at the concentration of $5 \times 10^4$ protoplasts/ml at 25°C in the dim for two weeks.

(3) Selection of transformed colonies:

Transformed colonies were selected in K3 culture medium containing 50 μg/ml kanamycin as reported by Hirofumi Uchimiya [Protein, Nucleic Acid and Enzyme, 30, 119 (1987)], and three weeks later 70 colonies of size greater than 2 mm in diameter were obtained. The colonies were transferred to K3 medium containing 100 μg/ml kanamycin, 30 colonies survived and increased their size to larger than 5 mm in diameter. Each of those colonies was clonally subcultured at 25°C in the light on MS medium containing 1 mg/l 1-naphthaleneacetic acid and 0.2 mg/l benzyladenine every other week. Two months later, ca. 10 g of callus was obtained from each colony. Each callus was subjected to Southern blot hybridization analysis.

Example 8: Detection of the transformed DNA by Southern blot hybridization.

(1) Plant DNA was isolated from the most rapidly growing callus obtained in Example 7 as described by J. Paszkowski [The EMBO Journal, 3 (12), 2717-2722 (1984)]. For NPT II gene detection, 30 μg of the isolated DNA was dissolved in MR buffer described in Example 4(1), and was digested with 100 units of HindIII and 100 units of EcoRI at 37°C for 2 hours. For detection of CP gene, 30 μg of the isolated DNA was dissolved in HR buffer described in Example 4(1), and was digested with 100 units of EcoRI and 100 units of XhoI at 37° C for 2 hours. Each reaction mixture was subjected to electrophoresis on 0.9%-horizontal agarose-gel as described in Example 2(9).

(2) The DNA fragments in the agarose gel obtained above in (1) as described by E.M. Southern (J. Mol. Biol., 98, 503-517 (1975),were transferred by capillary blotting onto a sheet of nylon membrane filter, Nytran (a commercial product of Schleicher & Schuell GMbH in West Germany) and the filter subjected to hybridization.

In the detection, autoradiography with Kodak X-Omat AR (a commercial product of EASTMAN KODAK COMPANY in U.S.A.) was performed at -70° C with intensifying screens. In consequence of autoradiography, when radiolabeled FRNPT mentioned below was used as a probe, the band corresponding to NTP II-gene fragment from the tobacco genome DNA digested by the used restriction enzymes was detected, and its size was calculated at ca.1.7 kbp. And, when radiolabeled BP-2 mentioned below is used as a probe, the band corresponding to CP-gene fragment from the tobacco genome DNA digested by the used restriction enzymes is detected, and its size is calculated at ca. 1.1 kbp.

(Preparation of radiolabeled probes)

5′-Terminal labeling method was used. To prepare the probe for the detection of NPTII-gene, 10 μg of the plasmid pNR constructed in Example 5 was dissolved in MR buffer, and was digested with 20 units of EcoRI and 20 units of HindIII at 37° C for 2 hours. To the reaction mixture, an equal volume of 2 M Tris-HCl buffer (pH 8.0) and 5 units of alkaline phosphatase were added, followed by dephosphorylation at 65° C for 30 minutes. The reaction mixture was deproteinized with phenol as described in Example 4(2) and mixture of DNA fragments was recovered by ethanol precipitation from the extract. The recovered mixture was subjected to electrophoresis on agarose gel, and the band corresponding to ca. 1.7 kbp DNA fragment was cut off. From the gel piece, 2 μg of 1,702 bp DNA fragment was eluted and purified. The DNA fragment dephosphorylated at 5′-termini was named FRNPT.

On the other hand, to prepare the probe for detection of CP-gene, non-phosphorylated 39-base oligonucleotide which was complementary to 5′-terminal of CP-gene, was synthesized by DNA synthesizer (model 380A which is a commercial product of Applied Biosystems Inc. in U.S.A.) and was named PB-2.

One μg of the probe FRNPT or PB-2 was dissolved in TK buffer [containing 50 mM Tris-HCl buffer (pH 7.6), 10 mM MgCl₂, 10 mM 2-mercaptoethanol], and was labeled at 5′-termini with 10 units of T4 polynucleotide kinase (produced by Takara Shuzo Co., Ltd., code: 2021a) and [γ-32p] ATP (3,000 Ci/m mol., a commercial product of Amersham Corporation in England, code: PB, 10218) at 37° C for 30 minutes. The unincorporated ATP was separated from the probe by gel filtration with Bio-gel P-30 (a commercial product of Bio-Rad Laboratories in U.S.A.), and the prove was recovered by ethanol precipitation.

Example 9: Preparation of linearized DNA fragments, NR-L and CCP-L

Fifty μg of plasmid pNR constructed in Example 5 was dissolved in HR buffer, and was digested with 150 units of PvuI (produced by Takara Shuzo Co., Ltd.) at 37° C for 2 hours. The reaction mixture was subjected to electrophoresis on agarose gel and the band corresponding to the larger DNA fragment was cut off. From the gel piece, 20 μg of 3,441 bp DNA fragment was eluted and purified. The resulting linearized DNA fragment was named NR-L. Fifty μg of the plasmed pCCP constructed in Example 6 was dissolved in HR buffer, and was digested with 150 units of PvuI at 37° C for 2 hours. The reaction mixture was subjected to electrophoresis on agarose gel. The band corresponding to the larger DNA fragment was cut off. From the gel piece, 22 μg of 3,364 bp DNA fragment was eluted and purified. The resulting linearized DNA fragment was named CCP-L. The obtained NR-L and CCP-L were subjected to electroporation as described in Example 7.

Example 10: Construction of pCP-NPT

Fifty μg of the plasmid pNR constructed in Example 5 was dissolved in MR buffer, and was digested with 120 units of HindIII at 37° C for 3 hours. Forty-eight μg of DNA fragment was recovered by deproteinization with a chloroform-phenol (1:1) mixture and ethanol precipitation. Forty-eight μg of the DNA fragment was dissolved in LF buffer [containing 67 mM potassium phosphate buffer (pH 7.4), 6.7 mM MgCl₂, 1 mM 2-mercaptoethanol], and was reacted with 50 units of Klenow fragment (a commercial product of Takara Shuzo Co., Ltd.) and 10 μl of dNTP solution as described in Example 4(1) at 37° C for 30 minutes to create blunt ends. Forty-five μg of the DNA fragment was recovered by deproteinization and ethanol precipitation. Forty-five μg of the DNA fragment and 0.05 O.D.unit of phosphorylated EcoRI linker (sequence: 5′ pCCGAATTCGG 3′, a commercial product of Takara Shuzo Co., Ltd., code: 4740P) were ligated by using the DNA ligation kit. Fivefold volume of HR buffer was added to the reaction mixture, followed by digestion with 200 units of EcoRI at 37°C for 2 hours. The reaction mixture was subjected to electrophoresis on agarose gel, and the band corresponding to ca. 1.7 kbp DNA fragment was cut off. From the gel piece, 8 μg of 1,714 bp DNA fragment, both termini of which were converted to EcoRi-protruding ends, was eluted and purified. The DNA fragment was cloned into the phosphatase-treated EcoRI site of the plasmid pCCP by using competent cells of E. coli JM109 which had been prepared as described in Example 2(7). From each colony, plasmid was isolated as described in Example 2(9). The clone harboring the plasmid affording ca. 1.7 kbp DNA fragment after digestion with EcoRI and ca. 1.6 kbp DNA fragment after digestion with KpnI respectively was selected to obtain a transformant (E. coli JM109/pCP-NPT). The resultant plasmid, which contained the kanamycin-resistance gene and the cp-gene on the same expression vector, was named pCP-NPT.

The plasmid pCP-NPT was subjected to electroporation as described in Example 7.

The transformant obtained in the above manner, namely Escherichia coli JM109/PCP-NPT, has been deposited at the Institute for Fermentation, Osaka (IFO) since February 5, 1988 under the deposit number IFO-14726 and also at the Fermentation Research Institute (FRI), Agency of Industrial Science and Technology, Ministry of International Trade and Industry since February 10, 1988 under the deposit number FERM BP-1729 .

Example 11: Plant regeneration from electroporated tobacco mesophyll protoplasts

Callus of size larger than 5 mm in diameter obtained in Example 7 were transferred to MS medium containing 0.5 mg/l benzyladenine (hereinafter abbreviated as BA) or 0.3 mg/l indole-3-acetic acid and 3 mg/l BA to induce shoot formation. Fully grown shoots were excised off from the callus and transferred to MS medium without plant growth regulators. Two weeks later, tobacco plants were obtained.

Example 12: Agrobacterium tumefaciens-mediated transformation of tobacco by using Ti-plasmid binary vector system

(1) One μg of the plasmid pBI101 in the kit of GUS GENE FUSION SYSTEM (produced by CLONTECH Laboratories, Inc. in U.S.A., code: #K1050) was dissolved in MR buffer, and was digested with 3 units of HindIII and 3 units of EcoRI at 37°C for 2 hours. After digestion, mixture of DNA fragments was recovered by deproteinization with chloroform-phenol (1:1) mixture and ethanol precipitation. The obtained mixture was named BIGUS-.

Ten μg of the plasmid pCAM35 obtained in Example 4, was dissolved in MR buffer, and was digested with 20 units of HindIII and 20 units of EcoRI at 37°C for 2 hours. The reaction mixture was subjected to electrophoresis on agarose gel, and the band corresponding to ca. 0.7 kbp DNA fragment was cut off. From the gel piece, 1.5 μg of DNA fragment, which contained the promoter- and terminator-region of CaMV, was eluted and purified. The resultant DNA fragment was named FRPT.

0.5 μg of BIGUS- and 1.5 μg of FRPT were ligated with the DNA ligation kit as described in Example 4(1), and the resultant circularized DNA was transformed into competent cells, which had been prepared from E. coli DH5α as described in Example 2(7), and was cloned by selecting with kanamycin. The resultant plasmid was named pBIC35

(2) Three μg of the plasmid pC2P constructed in Example 4(4) was dissolved in HR buffer, and was digested with 10 units of PstI at 37°C for 2 hours. From the reaction mixture, three μg of DNA fragment was recovered by ethanol precipitation. Three μg of the DNA fragment was dissolved in TP buffer, and was reacted with 10 units of T4 DNA polymerase and 10 μl of dNTP solution at 37°C for 30 minutes to create blunt ends. The DNA fragment recovered by ethaol precipitation was self-ligated, and was cloned. The resultant plasmid was named pC2P-.

Ten μg of the plasmid pC2P- was dissolved in MR buffer, and was digested with 20 units of HincII at 37°C for 2 hours. Ten μg of the DNA fragment was recovered by ethanol precipitation. Three μg of the DNA fragment and 0.05 O.D.unit of EcoRI linker described in Example 10 were ligated. Then fivefold volume of HR buffer was added to the reaction mixture, and the ligated fragments were digested with 50 units of EcoRI at 37°C for 2 hours. The reaction mixture was subjected to electrophoresis on agarose gel, and the band corresponding to the largest DNA fragment was cut off. From the gel piece, 9 μg of DNA fragment was eluted and purified as described in Example 4(2). The DNA fragment was cloned into the phosphatase-treated EcoRI site of pBIC35 obtained in (1), and the clone harboring the plasmid containing tandem-repeated CaMV promoters was selected. The resulting plasmid was named pBIC351. The 1,260 bp DNA fragment obtained in Example 6 (1) was cloned into the PstI-SmaI site of the plasmid pBIC351 to obtain a transformant E. coli DH 5α/pCCP32B1 (deposited at IFO since February 3, 1988 under the deposit No. IFO-14725 and also at FRI since February 10, 1988 under the deposit No. FERM BP- 1728). The resultant plasmid was named pCCP32B1.

(3) E.coli DH5α/pCCP32B1 (transformant harboring pCCP32B1) was pre-cultured on a plate (containing 15 g/l Bacto-agar, 10 g/l Bacto-tryptone, 5.0 g/l yeast extract, 0.58 g/l NaCl, 0.37 g/l MgSO₄•7H₂O, and 50 μg/ml kanamycin, pH7.5) at 30°C. E.coli HB101 harboring the helper plasmid (GUS GENE FUSION SYSTEM produced by CLONTECH Laboratories, Inc.) was pre-cultured on a LM plate (containing 50 μg/ml kanamycin in place of ampicillin in LM culture medium as described in Example 2(7)) at 30°C. Agrogacterium tumefaciens LBA4404 (GUS GENE FUSION SYSTEM produced by CLONTECH Laboratories, Inc.) was pre-cultured on a plate (containing 15 g/l Bacto-agar, 3.0 g/l K₂HPO₄, 1.0 g/l NaH₂PO₄, 1.0 g/l NH₄Cl, 0.3 g/l MgSO₄•7H₂O, 0.15 g/l KCl, 0.01 g/l CaCl₂, 2.5 mg/l FeSO₄•7H₂O, and 0.5 g/l glucose, pH7.0) at 28°C. These three kinds of colony described above were cultured together on a plate containing Nutrient-broth•hydrate (a commercial product of DIFCO LABORATORIES in U.S.A.) at 30°C for 2 days. The obtained mixture of colonies was transferred onto a selection plate (containing 15 g/l Bacto-agar, 3.0 g/l K₂HPO₄, 1.0 g/l NaH₂PO₄, 1.0 g/l NH₄Cl, 0.3 g/l MgSO₄•7H₂O, 0.15 g/l KCl, 0.01 g/l CaCl₂, 2.5 mg/l FeSO₄•7H2O and 50 μg/ml kanamycin), and was incubated at 30°C. Then A. tumefaciens harboring pCCP32B1 grew exclusively, and was cloned. The resultant clone was named A. tumefaciens LBA4404/pAL4404-pCCP32B1.

The transformant obtained in the above manner, namely A. tumefaciens LBA4404/pAL4404-pCCP32B1, has been deposited at the Institute for Fermentation, Osaka (IFO) since February 3, 1988 under the deposit number IFO-14722 and also at the Fermentation Research Institute (FRI), Agency of Industrial Science and Technology, Ministry of International Trade and Industry since February 10, 1988 under the deposit number FERM BP-1725.

(4) The leaf explants of tobacco (Nicotiana tabacum L.c.v. Bright yellow) are co-cultured with A. tumefaciens LBA4404/pAL4404-pCCP32B1 on a LS1 plate (Linsmaier & Skoog "LS" medium containing 10 g/l Bacto-agar, 2.0 mg/l 1-naphthaleneacetic acid (hereinafter abbreviated as NAA), and 0.2 mg/l BA) at 28°C for 48 hours, and subsequently are incubated at 26°C on a LS2 plate (LS medium containing 10 g/l Bacto-agar and 100 μg/ml carbenicillin) to remove A. tumefaciens. When the obtained cultures are transferred into LS4 medium (LS medium containing 0.1 mg/l NAA and 5.0 mg/l BA), shoots are induced. The resulting shoots are subjected to selection with LS4 medium containing 50 μg/ml of kanamycin, and then the object transformants are obtained.

Fig. 1 shows the base sequence (upper) of the cDNA obtained in Example 3 and the relevant amino acid sequence (lower), and Fig. 2 shows the electrophoretogram obtained in Example 2(1), with the fraction of RNA3 of CMV-Y being indicated.

## Claims

## Claims for the following Contracting States : AT, BE, CH, DE, FR, GB, GR, IT, LI, LU, NL, SE

1. A DNA which contains the segment of the base sequence of the coat protein of the cucumber mosaic virus strain Y shown in Fig. 1 which begins at position 418 and ends at position 1071.

2. The DNA of Claim 1 which comprises the segment of the base sequence shown in Fig. 1 which begins at position 418 and ends at position 1071, with at least one codon selected from the group consisting of TAA, TAG and TGA at the 3' end.

3. A DNA coding for a polypeptide corresponding to the segment of the amino acid sequence shown in Fig. 1 which begins with the 1st amino acid and ends in the 218th amino acid.

4. A vector DNA with a DNA according to claims 1-3 being inserted therein.

5. A bacterium, plant cell or plant transformed with a vector DNA with a DNA according to claims 1-3 being inserted therein.

## Claims for the following Contracting State : ES

1. A process for the production of a DNA which contains the segment of the base sequence of the coat protein of the cucumber mosaic virus strain Y shown in Fig. 1 which begins at position 418 and ends at position 1071 comprising
   - extracting RNA form CMV strain Y,
   - isolating RNA3 from total RNA,
   - synthesizing cDNA from RNA3 by means of reverse transcriptase,
   - cloning cDNA in a bacterial vector,
   - identifying said DNA.

2. The process of Claim 1 wherein the DNA is the segment of the base sequence shown in Fig. 1 which begins at position 418 and ends at position 1071, with at least one codon selected from the group consisting of TAA, TAG and TGA at the 3' end.

3. The process of claim 1 wherein the DNA codes for a polypeptide corresponding to the segment of the amino acid sequence shown in Fig. 1 wich begins with the 1st amino acid and ends in the 218th amino acid.

4. A process for the production of a vector DNA with a DNA according to claims 1 to 3 being inserted therein comprising inserting the DNA of claims 1 to 3 into a vector which is capable to transform a bacterium, a plant cell or a plant.

5. A process for the production of a bacterium, plant cell or plant transformed with a vector DNA with a DNA according to claims 1 to 3 being inserted therein comprising integrating said vector DNA by electroporation, Agrobacterium tumefaciens-mediated transformation, polyethylene glycol method, high pH-high calcium method, liposome method, microinjection method or a combination of any of such methods.

**Revendications**
**Revendications pour les Etats contractants suivants : AT, BE, CH, DE, FR, GB, GR, IT, LI, LU, NL, SE**

1. ADN qui contient, de la séquence de bases représentée sur la figure 1 et codant pour la protéine d'enveloppe de la souche Y du virus de la mosaïque du concombre, le segment qui commence en position 418 et se termine en position 1071.

2. ADN de la revendication 1, qui comporte, de la séquence de bases représentée sur la figure 1, le segment qui commence en position 418 et se termine en position 1071, avec au moins un codon choisi dans le groupe constitué par TAA, TAG et TGA à l'extrémité 3'-terminale.

3. ADN codant pour un polypeptide correspondant au segment de la séquence d'acides aminés, représentée sur la figure 1, qui commence avec le premier acide aminé et se termine avec le 218ème acide aminé.

4. ADN vecteur dans lequel est inséré un ADN conforme aux revendications 1 à 3.

5. Bactérie, cellule végétale ou plante transformée par un ADN vecteur dans lequel est inséré un ADN conforme aux revendications 1 à 3.

**Revendications pour l'Etat contractant suivant : ES**

1. Procédé de production d'un ADN contenant, de la séquence de bases, représentée sur la figure 1, codant pour la protéine d'enveloppe de la souche Y du virus de la mosaïque de concombre, le segment qui commence en position 418 et se termine en position 1071, lequel procédé comporte
   - l'extraction d'un ARN à partir de la souche Y de CMV
   - l'isolement de l'ARN-3, à partir de l'ARN total,
   - la synthèse d'un ADNc à partir de l'ARN-3 à l'aide d'une transcriptase inverse,
   - le clonage de l'ADNc dans un vecteur bactérien,
   - l'identification de cet ADN.

2. Procédé de la revendication 1, dans lequel l'ADN est le segment de la séquence de bases, représenté sur la figure 1, qui commence en position 418 et se termine en position 1071, avec, à l'extrémité 3'-terminale, au moins un codon choisi dans le groupe constitué par TAA, TAG et TGA.

3. Procédé de la revendication 1, dans lequel l'ADN code pour un polypeptide correspondant au segment de la séquence d'acides aminés, représentée sur la figure 1, qui commence avec le premier acide aminé et se termine avec le 218ème acide aminé.

4. Procédé de production d'un ADN vecteur dans lequel est inséré un ADN conforme aux revendications 1 à 3, comportant l'insertion de l'ADN des revendications 1 à 3 dans un vecteur qui est capable de transformer une bactérie, une cellule végétale ou une plante.

5. Procédé de production d'une bactérie, d'une cellule végétale ou d'une plante transformée à l'aide d'un ADN vecteur dans lequel est inséré un ADN conforme aux revendications 1 à 3, comprenant l'intégration dudit ADN vecteur par électroporation, transformation au moyen d'Agrobacterium tumefaciens, une méthode au polyéthylèneglycol, une méthode faisant intervenir un pH élevé et une teneur en calcium élevée, une méthode aux liposomes, une méthode de microinjection, ou une combinaison de n'importe lesquels de ces procédés.

**Patentansprüche**
**Patentansprüche für folgende Vertragsstaaten : AT, BE, CH, DE, FR, GB, GR, IT, LI, LU, NL, SE**

1. DNA, die das Segment der in Fig. 1 gezeigten Basissequenz des Hüllproteins des Gurkenmosaikvirus-Stammes Y enthält, das bei Position 418 beginnt und bei Position 1071 endet.

2. DNA nach Anspruch 1, die das Segment der in Fig. 1 gezeigten Basissequenz enthält, das bei Position 418 beginnt und bei Position 1071 endet, mit zumindest einem aus der aus TAA, TAG und TGA bestehenden Gruppe ausgewählten Kodon am 3'-Ende.

3. DNA, die für ein Polypeptid kodiert, das dem Segment der in Fig. 1 gezeigten Aminosäuresequenz entspricht, das mit der 1. Aminosäure beginnt und in der 218. Aminosäure endet.

4. Vektor-DNA mit einer darin eingefügten DNA nach Anspruch 1 bis 3.

5. Bakterie, Pflanzenzelle oder Pflanze, die mit einer Vektor-DNA mit einer darin eingefügten DNA nach Anspruch 1 bis 3 transformiert ist.

**Patentansprüche für folgenden Vertragsstaat : ES**

1. Verfahren zur Herstellung einer DNA, die das Segment der in Fig. 1 gezeigten Basissequenz des Hüllproteins des Gurkenmosaikvirus-Stammes Y enthält, das bei Position 418 beginnt und bei Position 1071 endet, umfassend
   - das Extrahieren von RNA aus CMV-Stamm Y
   - das Isolieren von RNA3 aus der gesamten RNA
   - das Synthetisieren von cDNA aus RNA3 durch Umkehrtranscriptase
   - das Klonen von cDNA in einem bakteriellen Vektor
   - das Identifizieren von genannter DNA.

2. Verfahren nach Anspruch 1, worin die DNA das Segment der in Fig. 1 gezeigten Basissequenz ist, das bei Position 418 beginnt und bei Position 1071 endet, mit zumindest einem aus der aus TAA, TAG und TGA bestehenden Gruppe ausgewählten Kodon am 3'-Ende.

3. Verfahren nach Anspruch 1, worin die DNA für ein Polypeptid kodiert, das dem Segment der in Fig. 1 gezeigten Aminosäuresequenz entspricht, das mit der 1. Aminosäure beginnt und in der 218. Aminosäure endet.

4. Verfahren zur Herstellung einer Vektor-DNA mit einer darin eingefügten DNA nach Anspruch 1 bis 3, welches das Einfügen der DNA nach Anspruch 1 bis 3 in einen Vektor umfaßt, der fähig ist, eine Bakterie, eine Pflanzenzelle oder eine Pflanze zu transformieren.

5. Verfahren zur Herstellung einer Bakterie, Pflanzenzelle oder Pflanze, die mit einer Vektor-DNA mit einer darin eingefügten DNA nach Anspruch 1 bis 3 transformiert ist, welches das Einfügen der genannten VektorDNA durch Elektroporation, Agrobakterien-tumefaciens-vermittelte Transformation, Polyethylenglycolverfahren, Hoch-pH-Hoch-Kalziumverfahren, Liposomverfahren, Mikroinjektionsverfahren oder eine Kombination jeglicher derartiger Verfahren umfaßt.

# Fig. 1-(1)

END NO. = 1370

EP 0 279 433 B1

```
   *    1                                                                    60*
CTGCA—GGG. GGG. GGG. GGA. ACA. GTG. AGT. CCG. AGG. AAT. TAA. ATG. TTG. AGA. GCC. CTC. CCG. CCG. CAA. TCG


   *   61                                                                   120*
GGA. GTT. CTT. CCG. CGT. CCC. GCT. CCG. AAG. CCT. TCA. GAC. CGC. AGG. TGG. TTA. ACG. GTC. TTT. AGC


   *  121                                                                   180*
ACT. TTG. GTG. CGT. ATT. AGT. ATA. TAA. GTA. TTT. GTG. AGT. CTT. TAC. ATA. ATA. CTA. TAT. CTA. TAG


   *  181                                                                   240*
TGT. CCT. GTG. TGA. GTT. GAT. ACA. GTA. GAC. ATC. TGT. GAC. GCG. ATG. CCG. TGT. TGA. GAA. GGG. AAC


   *  241                                                                   300*
ACA. TCT. GGT. TTT. AGT. AAG. CCT. ACA. TCA. TAG. TTT. TGA. GGT. TCA. ATT. CCT. CTT. ACT. CCC. TGT


   *  301                                                                   360*
TGA. GCC. CCC. TTA. CTT. TCT. CAT. GGA. TGC. TTC. TCC. GCG. AGA. TTG. CGT. TAT. TGT. CTA. CTG. ACT


   *  361                                                                   420*
ATA. TAG. AGA. GTG. TGT. GTG. CTG. TGT. TTT. CTC. TTT. TGT. GTC. GTA. GAA. TTG. AGT. CGA. GTC. ATG
                                                                                                 MET
```

Continued

# Fig. 1-(2)

```
*   421                                                                    480*
GAC. AAA. TCT. GAA. TCA. ACC. AGT. GCT. GGT. CGT. AAC. CGT. CGA. CGT. CGT. TTG. CGT. CGT. GGT. TCC
Asp-Lys-Ser-Glu-Ser-Thr-Ser-Ala-Gly-Arg-Asn-Arg-Arg-Arg-Arg-Leu-Arg-Arg-Gly-Ser

*   481                                                                    540*
CGC. TCC. GCC. TCC. TCC. TCC. TCG. GAT. GCT. AAC. TTT. AGA. GTC. TTG. TCG. CAG. CAG. CTT. TCG. CGA
Arg-Ser-Ala-Ser-Ser-Ser-Ser-Asp-Ala-Asn-Phe-Arg-Val-Leu-Ser-Gln-Gln-Leu-Ser-Arg

*   541                                                                    600*
CTT. AAC. AAG. ACG. TTA. GCA. GCT. GGT. CGT. CCA. ACT. ATT. AAC. CAC. CCA. ACC. TTT. GTA. GGG. AGT
Leu-Asn-Lys-Thr-Leu-Ala-Ala-Gly-Arg-Pro-Thr-Ile-Asn-His-Pro-Thr-Phe-Val-Gly-Ser

*   601                                                                    660*
GAA. CGC. TGT. AAA. CCT. GGG. TAC. ACG. TTC. ACA. TCT. ATT. ACC. CTA. AGG. CCA. CCA. AAA. ATA. GAC
Glu-Arg-Cys-Lys-Pro-Gly-Tyr-Thr-Phe-Thr-Ser-Ile-Thr-Leu-Arg-Pro-Pro-Lys-Ile-Asp

*   661                                                                    720*
CGT. GAG. TCT. TAT. TAT. GGT. AAA. AGG. TTG. TTA. TTA. CCT. GAT. TCA. GTC. ATG. GAA. TAT. GAT. AAG
Arg-Glu-Ser-Tyr-Tyr-Gly-Lys-Arg-Leu-Leu-Leu-Pro-Asp-Ser-Val-MET-Glu-Tyr-Asp-Lys

*   721                                                                    780*
AAG. CTT. GTT. TCG. CGC. ATT. CAA. ATT. CGA. GTT. AAT. CCT. TTG. CCG. AAA. TTT. GAT. TCT. ACC. GTG
Lys-Leu-Val-Ser-Arg-Ile-Gln-Ile-Arg-Val-Asn-Pro-Leu-Pro-Lys-Phe-Asp-Ser-Thr-Val
```

EP 0 279 433 B1

# Fig. 1-(3)

```
*   781                                                          840*
TGG. GTG. ACA. GTC. CGT. AAA. GTT. TCT. GCC. TCC. TCG. GAC. TTA. TCC. GTT. GCC. GCC. ATC. TCT. GCT
Trp-Val-Thr-Val-Arg-Lys-Val-Ser-Ala-Ser-Ser-Asp-Leu-Ser-Val-Ala-Ala-Ile-Ser-Ala

*   841                                                          900*
ATG. TTC. GCG. GAC. GGA. GCC. TCA. CCG. GTA. CTG. GTT. TAT. CAG. TAT. GCT. GCA. TCT. GGA. GTC. CAA
MET-Phe-Ala-Asp-Gly-Ala-Ser-Pro-Val-Leu-Val-Tyr-Gln-Tyr-Ala-Ala-Ser-Gly-Val-Gln

*   901                                                          960*
GCT. AAC. AAC. AAA. TTG. TTG. TAT. GAT. CTT. TCG. GCG. ATG. CGC. GCT. GAT. ATA. GGC. GAC. ATG. AGA
Ala-Asn-Asn-Lys-Leu-Leu-Tyr-Asp-Leu-Ser-Ala-MET-Arg-Ala-Asp-Ile-Gly-Asp-MET-Arg

*   961                                                         1020*
AAG. TAC. GCC. GTC. CTC. GTG. TAT. TCA. AAA. GAC. GAT. ACG. CTC. GAG. ACG. GAC. GAG. TTA. GTA. CTT
Lys-Tyr-Ala-Val-Leu-Val-Tyr-Ser-Lys-Asp-Asp-Thr-Leu-Glu-Thr-Asp-Glu-Leu-Val-Leu

*  1021                                                         1080*
CAT. GTT. GAC. GTC. GAG. CAC. CAA. CGC. ATT. CCC. ACA. TCT. GGA. GTG. CTC. CCA. GTC. TGA. TTC. CAT
His-Val-Asp-Val-Glu-His-Gln-Arg-Ile-Pro-Thr-Ser-Gly-Val-Leu-Pro-Val-***

*  1081                                                         1140*
GTT. CCC. AGA. ATC. CTC. CCT. CCG. ATC. TCT. GTG. GTG. GGA. GCT. GAG. TTG. GCA. GTT. CTG. CTA. TAA
```

Continued

EP 0 279 433 B1

# Fig. 1 –(4)

```
* 1141                                                                      1200*
ACT. GTC. TGA. AGT. CAC. TAA. ACG. TTT. TAC. GGT. GAA. CGG. GTT. GTC. CAT. CCA. GCT. TAC. GGC. TAA

* 1201                                                                      1260*
AAT. GGT. CAG. TCG. TGG. AGA. AAT. CTA. CGC. CAG. CAG. ATT. TAC. AAA. TCT. CTG. AGG. CGC. CTT. TGA

* 1261                                                                      1320*
AAC. CAT. CTC. CTA. GGT. TTC. TTC. GGA. AGG. ACT. TCG. GTC. CGT. GTA. CCT. CTA. GCA. CAA. CGT. GCT

* 1321
AGT. TTC. AGG. GTA. CGG. GTG. CCC. CCC. CAC. CTT. CGT. GGG. GCC. TCC. AAC. CCC. CCC. CCC — TGCAG
```

EP 0 279 433 B1

# Fig. 2

RNA 1

RNA 2

RNA 3

RNA 4

satellite RNA